# EUROPÄISCHE PATENTSCHRIFT

(11) **EP 2 699 614 B1**
(45) Veröffentlichungstag und Bekanntmachung des Hinweises auf die Patenterteilung: **14.01.2015**
(21) Anmeldenummer: 12714025.9
(22) Anmeldetag: 17.04.2012
(51) Int. Cl.: C08G 18/10, C08G 18/12, C08G 18/73, A61L 24/04

(54) **GEWEBEKLEBER MIT BESCHLEUNIGTER AUSHÄRTUNG**
CLOTH ADHESIVE WITH ACCELERATED HARDENING
ADHÉSIF TISSULAIRE AYANT UN DURCISSEMENT ACCÉLÉRÉ

(30) Priorität: 19.04.2011 EP 11162944
(43) Veröffentlichungstag der Anmeldung: 26.02.2014
(73) Patentinhaber: Medical Adhesive Revolution GmbH, 52074 Aachen (DE)
(72) Erfinder: HECKROTH, Heike, 51519 Odenthal (DE); EGGERT, Christoph, 50733 Köln (DE)
(74) Vertreter: Davepon, Björn
(86) Internationale Anmeldenummer: PCT/EP2012/057019
(87) Internationale Veröffentlichungsnummer: WO 2012/143358

(56) Entgegenhaltungen:
- EP-A1- 2 145 634
- EP-A1- 2 275 466

## Beschreibung

Die vorliegende Erfindung betrifft ein Polyharnstoff-System insbesondere zum Verschließen, Verbinden, Verkleben oder Abdecken von Zellgewebe sowie ein Dosiersystem für das erfindungsgemäße Polyharnstoff-System.

Diverse Materialien, die als Gewebekleber eingesetzt werden, sind im Handel erhältlich. Hierzu gehören die Cyanacrylate Dermabond^{®} (Octyl-2-Cyanoacrylat) und Histoacryl Blue^{®} (Butyl-Cyanoacrylat). Voraussetzung für eine effiziente Klebung der Cyanacrylate sind allerdings trockene Untergründe. Bei starken Blutungen versagen derartige Klebstoffe.

Als Alternative zu den Cyanacrylaten stehen biologische Klebstoffe wie zum Beispiel BioGlue^{®}, eine Mischung aus Glutaraldehyd und Bovinem Serumalbumin, diverse kollagen- und gelatinebasierte Systeme (FloSeal^{®}) sowie die Fibrinkleber (Tissucol) zur Verfügung. Diese Systeme dienen in erster Linie der Blutstillung (Hämostase). Neben den hohen Kosten zeichnen sich Fibrinkleber durch eine relative schwache Klebestärke und einen schnellen Abbau aus, so dass sie nur bei kleineren Verletzungen auf nicht gespanntem Gewebe verwendet werden können. Kollagen- und Gelatinebasierte Systeme wie FloSeal^{®} dienen ausschließlich der Hämostase. Zudem besteht, da Fibrin und Thrombin aus humanem-, Collagen und Gelatine aus tierischem Material gewonnen werden, bei biologischen Systemen immer die Gefahr einer Infektion. Biologische Materialien müssen außerdem gekühlt gelagert werden, so dass ein Einsatz in der Notfallversorgung wie z. B. in Katastrophengebieten, bei militärischen Einsetzen etc. nicht möglich ist. Hier steht zur Behandlung traumatischer Wunden QuikClot^{®} oder QuikClot ACS+™ zur Verfügung, welches ein mineralisches Granulat ist, das im Notfall in die Wunde gebracht wird und dort durch Wasserentzug zur Koagulation führt. Im Falle von QuikClot® ist dies eine stark exotherme Reaktion, die zu Verbrennungen führt. QuikClot ACS+™ ist eine Gaze, in die das Salz eingebettet ist. Das System muss zur Blutstillung fest auf die Wunde gedrückt werden.

Aus der EP 2 275 466 Al ist die Herstellung und Verwendung von Polyharnstoff-Systemen als Gewebekleber bekannt. Die hier offenbarten Systeme umfassen einen aminofunktionellen Asparaginsäureester und ein isocyanatfunktionelles Prepolymer. Zusätzlich ist ein tertiäres Amin enthalten. Dieses wird verwendet, um die Aushärtungsgeschwindigkeit des Polyharnstoff-Systems zu erhöhen, da dies insbesondere bei einer Verwendung zur Blutstillung von erheblicher Bedeutung ist. Die beschriebenen Polyharnstoff Systeme können als Gewebekleber für den Verschluss von Wunden in menschlichen und tierischen Zellverbänden eingesetzt werden. Dabei kann ein sehr gutes Klebeergebnis erzielt werden.

Allerdings besteht bei den in der EP 2 275 466 A1 beschriebenen Systemen die Gefahr, dass zumindest Teile der zur Beschleunigung der Aushärtung verwendeten Amine im Körper eluiert werden können. Hierdurch kann es zu unerwünschten biologischen Effekten kommen.

Aufgabe der Erfindung war es daher, ein Polyharnstoff-System mit besonders hoher Aushärtungsgeschwindigkeit bereit zu stellen, bei dem nicht die Gefahr einer Freisetzung von Aminen im Körper besteht.

Dieses Aufgabe ist erfindungsgemäß durch ein Polyhamstoff-System, umfassend
als Komponente A) isocyanatfunktionelle Prepolymere erhältlich durch Umsetzung von aliphatischen Isocyanaten A1) mit
   Polyolen A2), die insbesondere ein zahlenmittleres Molekulargewicht von ≥ 400 g/mol und eine mittlere OH-Funktionalität von 2 bis 6 aufweisen können,
als Komponente B) aminofunktionelle Verbindungen der allgemeinen Formel (I) 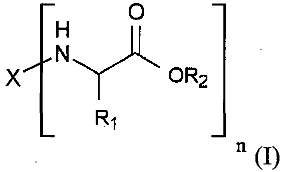 in der
   - X: ein organischer Rest enthaltend eine tertiäre Aminofunktion ist, der keinen Zerewitinoff-aktiven Wasserstoff aufweist,
   - R₁: ein CH₂-COOR₃-Rest, bei dem R₃ ein organischer Rest ist, der keinen Zerewitinoff-aktiven Wasserstoff aufweist, ein linearer oder verzweigter C1-bis C4-Alkyl-, ein Cyclopentyl-, ein Cyclohexyl-Rest oder H ist,
   - R₂: ein organischer Rest ist, der keinen Zerewitinoff-aktiven Wasserstoff aufweist,
   - n: 2 oder 3 ist,
gelöst.

Das erfindungsgemäße Polyharnstoff-System zeichnet sich durch eine sehr hohe Aushärtungsgeschwindigkeit aus, wodurch es sich insbesondere zur Blutungsstillung eignet. Da das erfindungsgemäße System auch keine nicht ins polymere Netzwerk eingebundenen niedermolekularen Amine enthält, besteht auch nicht die Gefahr, dass derartige Verbindungen eluiert und damit im Körper freigesetzt werden könnten.

Zur Definition von Zerewitinoff-aktivem Wasserstoff wird auf Römpp Chemie Lexikon, Georg Thieme Verlag Stuttgart verwiesen. Bevorzugt werden unter Gruppen mit Zerewitinoff-aktivem Wasserstoff OH, NH oder SH verstanden.

Gemäß einer bevorzugten Ausführungsform des erfindungsgemäßen Polyharnstoff-Systems ist X ein Rest der Formel (II) 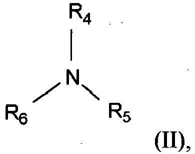 in der
- R₄, R₅, R₆: jeweils unabhängig voneinander ein organischer Rest sind, der keinen Zerewitinoffaktiven Wasserstoff aufweist.

Besonders bevorzugt ist hier, wenn R₄, R₅, R₆ jeweils unabhängig voneinander oder gleichzeitig ein linearer oder verzweigter, gegebenenfalls auch in der Kette mit Heteroatomen substituierter gesättigter organischer Rest, insbesondere ein linearer oder verzweigter, gesättigter, aliphatischer C1 bis C10, bevorzugt C2 bis C8 und besonders bevorzugt C2 bis C6 Kohlenwasserstoffrest sind.

Ganz besonders bevorzugt sind R₄, R₅, R₆ jeweils unabhängig voneinander oder gleichzeitig ein Methyl, Ethyl, Propyl- oder Butyl-Rest.

Ebenfalls vorteilhaft ist ein Polyharnstoff-System umfassend eine Verbindung der Formel (I) in der die Reste R₁, R₂ und gegebenenfalls R₃ jeweils unabhängig voneinander oder gleichzeitig ein linearer oder verzweigter C1 bis C 10, bevorzugt C1 bis C8, besonders bevorzugt C2 bis C6, ganz besonders bevorzugt C2 bis C4 organischer Rest und insbesondere ein aliphatischer Kohlenwasserstoffrest sind. Beispiele besonders geeigneter Reste sind Methyl, Ethyl, Propyl und Butyl.

Die isocyanatfunktionellen Prepolymere A) sind durch Umsetzung von Polyisocyanaten A1) mit Polyolen A2) gegebenenfalls unter Zusatz von Katalysatoren sowie Hilfs- und Zusatzstoffen erhältlich.

Als Polyisocyanate A1) können beispielsweise monomere aliphatische oder cycloaliphatische Di- oder Triisocyanate wie 1,4-Butylendiisocyanat (BDI), 1,6-Hexamethylendiisocyanat (HDI), Isophorondiisocyanat (IPDI), 2,2,4- und/oder 2,4,4-Trimethylhexa-methylendiisocyanat, die isomeren Bis-(4,4'-isocyanatocyclohexyl)-methane oder deren Mischungen beliebigen Isomerengehalts, 1,4-Cyclohexylendiisocyanat, 4-Isocyanatomethyl-1,8-octandiisocyanat (Nonan-triisocyanat), 2-Isocyanatoethyl-6-isocyanatocaproat, sowie Alkyl-2,6-diisocyanatohexanoat (Lysindiisocyanat) mit C1-C8-Alkylgruppen eingesetzt werden.

Neben den vorstehend genannten monomeren Polyisocyanaten A1) können auch deren höhermolekulare Folgeprodukte mit Uretdion-, Isocyanurat-, Urethan-, Allophanat-, Biuret-, Iminooxadiazindion- oder Oxadiazintrionstruktur sowie deren Mischungen verwendet werden.

Bevorzugt werden Polyisocyanate A1) der vorstehend genannten Art mit ausschließlich aliphatisch oder cycloaliphatisch gebundenen Isocyanatgruppen oder deren Mischungen eingesetzt.

Ebenfalls bevorzugt ist, wenn Polyisocyanate A1) der vorstehenden Art mit einer mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 verwendet werde.

Ganz besonders bevorzugt wird Hexamethylendiisocyanat als Polyisocyanat A1) eingesetzt,

Gemäß einer weiteren bevorzugten Ausführungsform des erfindungsgemäßen Polyharnstoff-Systems ist vorgesehen, dass die Polyole A2) Polyesterpolyole und / oder Polyester-Polyether-Polyole und / oder Polyetherpolyole sind. Insbesondere bevorzugt sind dabei Polyester-Polyether-Polyole und / oder Polyetherpolyole mit einem Ethylenoxidanteil zwischen 60 und 90 Gew.-%.

Bevorzugt ist auch, wenn die Polyole A2) ein zahlenmittleres Molekulargewicht von 4000 bis 8500 g/mol aufweisen.

Geeignete Polyetheresterpolyole werden entsprechend dem Stand der Technik vorzugsweise durch Polykondensation aus Polycarbonsäuren, Anhydriden von Polycarbonsäuren, sowie Estern von Polycarbonsäuren mit leichtflüchtigen Alkoholen, bevorzugt C1 bis C6 Monoolen, wie Methanol, Ethanol, Propanol oder Butanol, mit molar überschüssigem, niedermolekularem und/oder höhermolekularem Polyol hergestellt; wobei als Polyol ethergruppenhaltige Polyole gegebenenfalls in Mischungen mit anderen ethergruppenfreien Polyolen eingesetzt werden.

Selbstverständlich können zur Polyetherestersynthese auch Gemische der höhermolekularen und der niedermolekularen Polyole verwendet werden.

Solche molar überschüssigen niedermolekularen Polyole sind Polyole mit Molmassen von 62 bis 299 Dalton, mit 2 bis 12 C-Atomen und Hydroxylfunktionalitäten von mindestens 2, die weiterhin verzweigt oder unverzweigt sein können und deren Hydroxylgruppen primär oder sekundär sind. Diese niedermolekularen Polyole können auch Ethergruppen aufweisen. Typische Vertreter sind Ethylenglykol, Propandiol-1,2, Propandiol-1,3, Butandiol-1,4, Butandiol-2,3, 2-Methyl-propandiol-1,3, Pentandiol-1,5, Hexandiol-1,6,3-Methyl-pentandiol-1,5, 1,8-Octandiol, 1,10-Decandiol, 1,12-Dodecandiol, Cyclohexandiol, Diethylenglykol, Triethylenglykol und höhere Homologe, Dipropylenglykol, Tripropylenglykol und höhere Homologe, Glycerin, 1,1,1-Trimethylolpropan, sowie Oligo-tetrahydrofurane mit Hydroxylendgruppen. Selbstverständlich können innerhalb dieser Gruppe auch Gemische verwendet werden.

Molar überschüssige höhermolekulare Polyole sind Polyole mit Molmassen von 300 bis 3000 Dalton, die durch ringöffenende Polymerisation von Epoxiden, bevorzugt Ethylen- und/oder Propylenoxid, sowie durch säurekatalysierte, ringöffnende Polymerisation von Tetrahydrofuran, erhalten werden können. Zur ringöffnenden Polymerisation von Epoxiden können entweder Alkalihydroxide oder Doppelmetallcyanidkatalysatoren verwendet werden.

Als Starter für ringöffnende Epoxidpolymerisationen können alle mindestens bifunktionellen Moleküle aus der Gruppe der Amine und der o.g. niedermolekularen Polyole verwendet werden. Typische Vertreter sind 1,1,1-Trimethylolpropan, Glycerin, o-TDA, Ethylendiamin, Propylenglykol-1,2, etc. sowie Wasser, einschließlich deren Gemische. Selbstverständlich können innerhalb der Gruppe der überschüssigen höhermolekularen Polyole auch Gemische verwendet werden.

Der Aufbau der höhermolekularen Polyole, soweit es sich um Hydroxylgruppen terminierte Polyalkylenoxide aus Ethylen- und/oder Propylenoxid handelt, kann statistisch oder blockweise erfolgen, wobei auch Mischblöcke enthalten sein können.

Polycarbonsäuren sind sowohl aliphatische als auch aromatische Carbonsäuren, die sowohl cyclisch, linear, verzweigt oder unverzweigt sein können und die zwischen 4 und 24 C-Atome aufweisen können.

Beispiele sind Bernsteinsäure, Glutarsäure, Adipinsäure, Azelainsäure, Sebacinsäure, 1,10-Decandicarbonsäure, 1,12-Dodecandicarbonsäure, Phthalsäure, Terephthalsäure, Isophthalsäure, Trimellitsäure, Pyromellitsäure. Bevorzugt sind Bernsteinsäure, Glutarsäure, Adipinsäure, Sebacinsäure, Milchsäure, Phthalsäure, Terephthalsäure, Isophthalsäure, Trimellitsäure, Pyromellitsäure. Besonders bevorzugt sind Bernsteinsäure, Glutarsäure und Adipinsäure.

Weiterhin umfasst die Gruppe der Polycarbonsäuren auch Hydroxycarbonsäuren, bzw. deren innere Anhydride, wie z.B. Caprolacton, Milchsäure, Hydroxybuttersäure, Ricinolsäure, usw. Mit umfasst sind weiterhin auch Monocarbonsäuren, insbesondere solche, die über mehr als 10 C-Atome verfügen, wie Sojaölfettsäure, Palmölfettsäure und Erdnussölfettsäure, wobei deren Anteil am gesamten, das Polyetheresterpolyol aufbauenden Reaktionsmischung 10 Gew.-% nicht übersteigt und zusätzlich die dadurch einhergehende Minderfunktionalität durch Mitverwendung von mindestens trifunktionellen Polyolen, sei es auf Seiten der niedermolekularen oder der hochmolekularen Polyole ausgeglichen wird.

Die Herstellung der Polyetheresterpolyol erfolgt bevorzugt entsprechend dem Stand der Technik bei erhöhter Temperatur im Bereich von 120 bis 250 °C, zunächst unter Normaldruck, später unter Anlegen von Vakuum von 1 bis 100 mbar, vorzugsweise, aber nicht notwendigerweise unter Verwendung eines Veresterungs- oder Umesterungskatalysators, wobei die Reaktion so weit vervollständigt wird, dass die Säurezahl auf Werte von 0,05 bis 10 mg KOH/g, bevorzugt 0,1 bis 3 mg KOH/g und besonders bevorzugt 0,15 bis 2,5 mg KOH/g absinkt.

Weiterhin kann im Rahmen der Normaldruckphase vor dem Anlegen von Vakuum ein Inertgas verwendet werden. Selbstverständlich können alternativ oder für einzelne Phasen der Veresterung auch flüssige oder gasförmige Schleppmittel zum Einsatz kommen. Beispielsweise kann das Reaktionswasser unter Verwendung von Stickstoff als Trägergas, ebenso ausgetragen werden, wie unter Einsatz eines Azeotropschleppmittels, wie z.B. Benzol, Toluol, Xylol, Dioxan, etc.

Selbstverständlich können auch Abmischungen von Polyetherpolyolen mit Polyesterpolyolen in beliebigen Verhältnissen eingesetzt werden.

Polyetherpolyole sind bevorzugt Polyalkylenoxid-Polyether auf Basis von Ethylenoxid und gegebenenfalls Propylenoxid.

Diese Polyetherpolyole basieren bevorzugt auf di- oder höherfunktionellen Startermolekülen wie zwei- oder höherfunktionellen Alkoholen oder Aminen.

Beispiele solcher Starter sind Wasser (als Diol aufgefasst), Ethylenglykol, Propylenglykol, Butylenglykol, Glycerin, TMP, Sorbit, Pentaerythrit, Triethanolamin, Ammoniak oder Ethylendiamin.

Ebenfalls können Hydroxylgruppen aufweisende Polycarbonate, bevorzugt Polycarbonatdiole, mit zahlenmittleren Molekulargewichten Mₙ von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art in Frage.

Zur Herstellung des Prepolymers A) kann das Polyisocyanat A1) mit dem Polyol A2) bei einem NCO/OH-Verhältnis von bevorzugt 4:1 bis 12:1, besonders bevorzugt 8:1 umgesetzt und anschließend der Anteil an nicht umgesetzten Polyisocyanates mittels geeigneter Methoden abgetrennt werden. Üblicherweise wird hierfür die Dünnschichtdestillation verwendet, wobei Prepolymere mit Restmonomergehalten von weniger als 1 Gew.-%, bevorzugt weniger als 0,1 Gew.-%, ganz besonders bevorzugt weniger als 0,03 Gew.-% erhalten werden.

Gegebenenfalls können während der Herstellung Stabilisatoren wie Benzoylchlorid, Isophthaloylchlorid, Dibutylphosphat, 3-Chlorpropionsäure oder Methyltosylat zugesetzt werden.

Die Reaktionstemperatur bei der Herstellung der Prepolymere A) beträgt dabei bevorzugt 20 bis 120 °C und weiter bevorzugt 60 bis 100 °C.

Die hergestellten Prepolymere haben einen nach DIN EN ISO 11909 gemessenen mittleren NCO-Gehalt von 2 bis 10 Gew.-%, bevorzugt 2,5 bis 8 Gew.-%.

Gemäße einer weiteren Ausführungsform des erfindungsgemäßen Polyharnstoff Systems können die Prepolymere A) eine mittlere NCO-Funktionalität von 1,5 bis 2,5, bevorzugt von 1,6 bis 2,4, weiter bevorzugt von 1,7 bis 2,3, ganz besonders bevorzugt von 1,8 bis 2,2 und insbesondere von 2 aufweisen.

In Weiterbildung der Erfindung ist vorgesehen, dass das Polyharnstoff-System zusätzlich organische Füllstoffe C) umfasst. Diese können insbesondere eine nach DIN 53019 gemessenen Viskosität bei 23 °C im Bereich von 10 bis 20.000 mPas, bevorzugt von 50 bis 4000 mPas und besonders bevorzugt von 50 bis 2000 mPas aufweisen.

Bei den organischen Füllstoffen der Komponente C) kann es sich vorzugsweise um hydroxyfunktionelle Verbindungen, insbesondere um Polyetherpolyolehandeln.

Vorteilhaft ist auch, wenn die Füllstoffe der Komponente C) eine mittlere OH-Funktionalität von 1,5 bis 3, bevorzugt von 1,8 bis 2,2 und besonders bevorzugt von 2 aufweisen.

Beispielsweise können als organische Füllstoffe C) bei 23 °C flüssige Polyethylenglykole wie PEG 200 bis PEG 600, deren Mono- bzw. Dialkylether wie PEG 500 Dimethylether, flüssige Polyether- und Polyesterpolyole, flüssige Polyester wie z.B. Ultramoll (Lanxess AG, Leverkusen, DE) sowie Glycerin und seine flüssigen Derivate wie z.B. Triacetin (Lanxess AG, Leverkusen, DE) eingesetzt werden.

In einer weiteren bevorzugten Ausführungsform des erfindungsgemäßen Polyharnstoff-Systems werden als organische Füllstoffe Polyethylenglykole eingesetzt. Diese haben vorzugsweise ein zahlenmittleres Molekulargewicht von 100 bis 1000 g/mol und besonders bevorzugt von 200 bis 400 g/mol.

Um das mittlere Equivalentgewicht der insgesamt zur Prepolymervernetzung eingesetzten Verbindungen bezogen auf die NCO-reaktiven Gruppen weiter zu reduzieren, ist es möglich zusätzlich Umsetzungsprodukte der Prepolymere A) mit der aminofunktionellen Verbindung B) und / oder den organischen Füllstoffen C), sofern diese amino- oder hydroxyfunktionell sind, in einer separaten Vorreaktion herzustellen und dann als höhermolekulare Härterkomponente einzusetzen.

Bevorzugt werden bei der Vorverlängerung Verhältnisse von isocyanatreaktiven Gruppen zu Isocyanatgruppen von 50 zu 1 bis 1,5 zu 1, besonders bevorzugt 15 zu 1 bis 4 zu 1 eingestellt.

Vorteil dieser Modifizierung durch Vorverlängerung ist, dass das Equivalentgewicht und das Equivalentvolumen der Härterkomponente in größeren Grenzen modifizierbar ist. Dadurch können zur Applikation kommerziell verfügbare 2-Kammerdosiersysteme eingesetzt werden, um ein System zu erhalten, das bei bestehenden Verhältnissen der Kammervolumina in das gewünschte Verhältnis von NCO-reaktiven Gruppen zu NCO-Gruppen eingestellt werden kann.

Selbstverständlich können in die Polyharnstoff-Systeme auch pharmakologisch aktive Wirkstoffe wie Analgetika mit und ohne antiinflammatorische Wirkung, Antiphlogistika, antimikrobiell wirksame Substanzen, Antimykotika, antiparasitär wirkende Stoffe als Komponente D) eingebracht werden.

Das erfindungsgemäße Polyharnstoff-System kann durch Mischung der Prepolymere A) mit den aminofunktionellen Verbindung B) sowie gegebenenfalls den Komponenten C) und D) erhalten werden. Das Verhältnis von freien oder blockierten Aminogruppen zu freien NCO-Gruppen beträgt dabei bevorzugt 1:1,5, besonders bevorzugt 1:1.

Das erfindungsgemäße Polyharnstoff-System ist besonders zum Verschließen, Verbinden, Verkleben oder Abdecken von Zellgewebe und insbesondere zur Stillung des Austritts von Blut oder Gewebeflüssigkeiten oder dem Verschluss von Leckagen in Zellgewebe geeignet. Ganz besonders bevorzugt kann es zur Verwendung oder zur Herstellung eines Mittels zum Verschließen, Verbinden, Verkleben oder Abdecken von menschliches oder tierisches Zellgewebe verwendet werden. Mit seiner Hilfe können schnell aushärtende, stark am Gewebe anhaftende, transparente, flexible und biokompatible Klebnähte hergestellt werden.

Noch ein weiterer Gegenstand der Erfindung ist Dosiersystem mit zwei Kammern für ein erfindungsgemäßes Polyharnstoff-System, bei dem in der einen Kammer die Komponente A) und in der anderen Kammer die Komponenten B) und gegebenenfalls die Komponenten C) und. D) des Polyharnstoff-Systems enthalten sind. Ein derartiges Dosiersystem eignet sich insbesondere dafür das Polyharnstoff-System als Kleber auf Gewebe zu applizieren.

Die Erfindung wird im Folgenden anhand von Beispielen näher erläutert.

### Beispiele:

### Methoden:

- Molekulargewichte: wurden mittels Gelpermeationschromatographie (GPC) wie folgt bestimmt: Die Kalibrierung erfolgt mit Polystyrol-Standards mit Molekulargewichten von Mp 1.000.000 bis 162. Als Eluent wurde Tetrahydrofuran p.A. verwendet. Die folgenden Parameter wurden bei der Doppelmessung eingehalten: Entgasung: Online - Degasser; Durchfluß: 1 ml/Min; Analysenzeit: 45 Minuten; Detektoren: Refraktometer und UV-Detektor; Injektionsvolumen: 100 µl - 200 µl. Die Berechnung der Molmassenmittelwerte Mw; Mn und Mp sowie der Polydispersität Mw/Mn erfolgte softwaregestützt. Basislinienpunkte und Auswertegrenzen wurden entsprechend der DIN 55672 Teil 1 festgelegt.
- NCO-Gehalte: wurden, wenn nicht ausdrücklich anders erwähnt, volumetrisch gemäß DIN-EN ISO 11909 bestimmt.
- Viskositäten: wurde nach ISO 3219 bei 23 °C bestimmt.
- Restmonomergehalte: wurden nach DIN ISO 17025 bestimmt.
- NMR-Spektren: Wurde mit einem Bruker DRX 700 Gerät bestimmt.

### Substanzen:

- HDI:: Hexamethylendiisocyanat (Bayer MaterialScience AG)

Alle anderen Chemikalien wurden bei den Firmen Aldrich und Fluka bezogen.

### Synthese von Triethyl-11-methyl-4-oxo-3-oxa-7,11,15-triazaheptadecan-6,16,17-tricarboxylat (1)

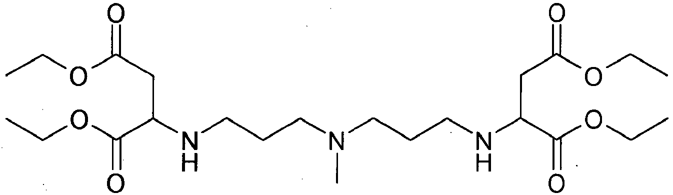

Zu 8,7 g (0,06 Mol) N,N'Bis-(aminopropyl)methylamin wurden 20,66 g (0,12 Mol) Maleinsäurediethylester gegeben. Die Reaktionsmischung wurde 3 Tage bei 60 °C gerührt. Das Produkt wurde quantitativ als gelbe Flüssigkeit erhalten.

¹H-NMR (CDCl₃, 700 MHz): δ = 1.29 (t, 12H), 1.67 (t, 4H), 1,89 (br, 2NH), 2,2 (s, 3H) 2,34 (t, 4H), 2,51 (m, 2H), 2,6 (m, 2H), 2,7 (m, 4H), 3.60 (t, 2H), 4.13 (q, 4H), 4.18 (q, 4H).

¹³C-NMR (CDCl₃, 700 MHz): 13.9, 27,5, 37.9, 41.9, 46.3, 55.6, 57.6, 60.4, 60.7, 170.6, 173.4.

### Synthese von Triethyl-4-oxo-3-oxa-7,14,21-triazatricosan-6,22,23-tricarboxylat (2)

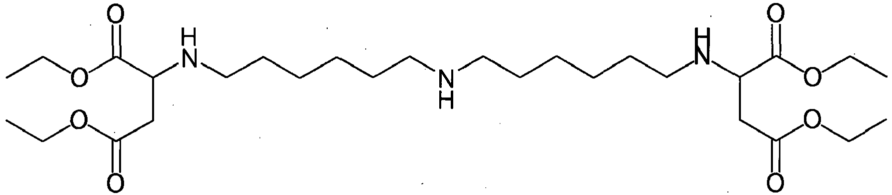

Analog zu (2) wurden aus 154 g (0,72 Mol) Bis(hexamethylen)-triamin und 246 g (1,42 Mol) Maleinsäurediethylester 400 g des Produktes als gelbe Flüssigkeit erhalten.

¹H-NMR (CDCl₃, 700 MHz): δ = 1.27 (t, 6H), 1,29 (t, 6H), 1.34 (m, 8H), 1.49 (br, 3NH), 2,59 (m, 12H), 3.70 (t, 2H), 4.11 (q, 4H), 4.2 (q, 4H).

¹³C-NMR (CDCl₃, 700 MHz): 14.1, 26.8, 27.1, 30.0, 30.1, 38.1, 47.9, 50.1, 57.8, 60.3, 60.8, 170.7, 172.7.

### Tetraethyl-2,2'-[(2-methylpentan-1,5-diyl)diimino]dibutandioat (3)

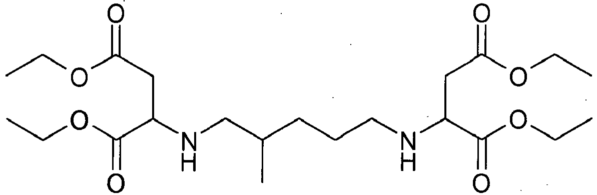

Zu 2 Mol Diethylmaleat wurde unter Stickstoffatmosphäre langsam 1 Mol 2-Methyl-1,5-diaminopentan getropft, so dass die Reaktionstemperatur 60°C nicht überschritt. Anschließend wurde so lange auf 60°C erwärmt, bis kein Diethylmaleat mehr im Reaktionsgemisch nachweisbar war. Das Produkt wurde quantitativ als gelbe Flüssigkeit erhalten.

¹H-NMR (CDCl₃, 700 MHz): δ = 0.89 (d, 3H), 1.11 (m, 1H), 1.27 (t, 6 H), 1.32 (t, 6H), 1.4 (m, 4H), 1.5 (br, 2NH), 2.51 (m, 8H), 3.6 (m, 2H), 4.18 (m, 4H), 4.26 (q, 4H),

### Synthese von Prepolymer A

465 g HDI und 2.35 g Benzoylchlorid wurden in einem 11 Vierhalskolben vorgelegt. Innerhalb von 2 h wurden bei 80°C 931.8 g eines trifunktionellen Polyethers der Molmasse 4500, gestartet auf Glycerin und einem Ethylenoxidgehalt von 71 % und einem Propylenoxidgehalt von 29 %, jeweils bezogen auf dem gesamten Alkylenoxidgehalt, hinzugefügt und 1 h nachgerührt. Anschließend wurde durch Dünnschichtdestillation bei 130 °C und 0,13 mbar das überschüssige HDI abdestilliert. Man erhielt 980 g (71 %) des Prepolymers mit einem NCO-Gehalt von 2,37 % und einer Viskosität von 4500 mPas/23°C. Der Restmonomerengehalt betrug < 0,03 % HDI.

### Synthese von Prepolymer B

263 g (1,8 mol) Adipinsäure wurden mit 1591,5 g Polyethylenglykol 600 (2,6 mol) unter Rühren auf 235 °C erhitzt. Dabei wurde über 8,5 h das entstehende Wasser abdestilliert. Anschließend wurden 100 ppm Zinn(II)chlorid hinzugegeben und für weitere 9 h unter Vakuum (15 mbar) am Wasserabscheider auf 235°C erhitzt.

672 g HDI (4 mol) wurden mit 0,1 Gew% Benzoylchlorid vorgelegt und auf 80 °C erhitzt. Anschließend wurden unter Rühren 788 g des zuvor hergestellten Polyesters über 1h zudosiert und bis zum Erreichen eines konstanten NCO-Gehalts bei 80 °C weiter gerührt. Das überschüssige HDI wurde bei 140 °C und 0,13 mbar mittels Dünnschichtverdampfer entfernt. Das erhaltene Prepolymer hatte einen NCO-Gehalt von 3,5 % und eine Viskosität von 4700 mPas/23°C. Der Restmonomerengehalt betrug < 0,03 % HDI.

### Herstellung des Gewebeklebers

4 g des jeweiligen Prepolymers wurden mit einer equivalenten Menge der Mischung aus 1 mit dem in BMS 111002 beschriebenen Triethyl-4-oxo-3-oxa-7,14,21-triazatricosan-6,22,23-tricarboxylat 2 beziehungsweise mit Tetraethyl-2,2'-[(2-methylpentan-1,5-diyl)diimino]dibutandioat 3 in einem Becher gut verrührt. Das Polyharnstoff-System wurde unmittelbar danach auf das zu klebende Muskelgewebe als dünne Schicht aufgetragen. Als Verarbeitungszeit wurde dabei die Zeit bestimmt, innerhalb der das Klebstoff-System noch eine so niedrige Viskosität besaß, dass es problemlos auf das Gewebe aufgetragen werden konnte.

| | Härter | Verhältnis der Härter | Verarbeitungszeit [s] |
|---|---|---|---|
| Prepolymer A (HXH 100) | **3 pur** | / | 240 |
| Prepolymer A | **3 mit 1** | 0,9:0,1 | 108 |
| Prepolymer A | **3 mit 1** | 0,80:0,20 | 60 |
| Prepolymer A | **3 mit 1** | 0,70:0,30 | 45 |
| Prepolymer A | **3 mit 1** | 0,60:0,40 | 20 |
| Prepolymer A | **3 mit 1** | 0,50:0,50 | 20 |
| Prepolymer B | **2** | / | 120 |
| Prepolymer B | **2 mit 1** | 0,90:0,10 | 82 |
| Prepolymer B | **2 mit 1** | 0,80:0,20 | 60 |
| Prepolymer B | **2 mit 1** | 0,70:0,30 | 35 |
| Prepolymer B | **2 mit 1** | 0,60:0,40 | 29 |
| Prepolymer B | **2 mit 1** | 0,50:0,50 | 20 |

Die in der vorstehenden Tabelle aufgeführten Ergebnisse zeigen, dass die erfindungsgemäßen Polyharnstoff-Systeme eine sehr hohe Aushärtungsgeschwindigkeit aufweisen. Darüber hinaus besitzen die Systeme auch eine gute Klebleistung. Da die erfindungsgemäßen Systeme auch keine nicht ins polymere Netzwerk eingebundenen niedermolekularen Amine enthalten, besteht auch nicht die Gefahr, dass derartige Verbindungen eluiert und damit im Körper freigesetzt werden könnten.

## Patentansprüche

1. Polyharnstoff-System, umfassend
als Komponente A) isocyanatfunktionelle Prepolymere erhältlich durch Umsetzung von aliphatischen Isocyanaten A1) mit
Polyolen A2),
als Komponente B) aminofunktionelle Verbindungen der allgemeinen Formel (I) 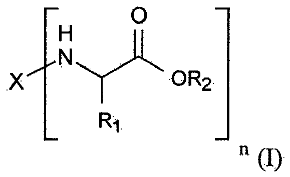 in der
X ein organischer Rest enthaltend eine tertiäre Aminofunktion ist, der keinen Zerewitinoff-aktiven Wasserstoff aufweist,
R₁ ein CH₂-COOR₃-Rest, bei dem R₃ ein organischer Rest ist, der keinen Zerewitinoff-aktiven Wasserstoff aufweist, ein linearer oder verzweigter C1-bis C4-Alkyl-, ein Cyclopentyl-, ein Cyclohexyl-Rest oder H ist,
R₂ ein organischer Rest ist, der keinen Zerewitinoff-aktiven Wasserstoff aufweist,
n 2 oder 3 ist.

2. Polyharnstoff-System nach Anspruch 1, **dadurch gekennzeichnet, dass** X ein Rest der Formel (II) 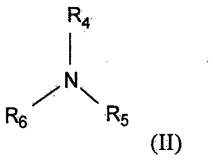 ist, in der
R₄, R₅, R₆ jeweils unabhängig voneinander ein organischer Rest sind, der keinen Zerewitinoff-aktiven Wasserstoff aufweist.

3. Polyharnstoff-System nach Anspruch 2, **dadurch gekennzeichnet, dass** R₄, R₅, R₆ jeweils unabhängig voneinander oder gleichzeitig ein linearer oder verzweigter, sind.

4. Polyharnstoff-System nach Anspruch 3, dadurch gekenntzeichnet, dass R₄, R₅, R₆ jeweils unabhängig voneinander oder gleichzeitig ein Methyl, Ethyl, Propyl- oder Butyl-Rest sind, wobei wenigstens einer der Reste R₄, R₅, R₆ ein Methylen-, Ethylen-, Propylen-oder Butylen-Rest ist.

5. Polyharnstoff-System nach einem der Ansprüche 1 bis 4, **dadurch gekennzeichnet, dass** die Reste R₁, R₂ und gegebenenfalls R₃ jeweils unabhängig voneinander oder gleichzeitig ein linearer oder verzweigter C1 bis C 10 organischer Rest sind.

6. Polyharnstoff-System nach einem der Anspruche 1 bis 5, **dadurch gekennzeichnet, dass** die Polyole A2) Polyesterpolyole und / oder Polyester-Polyether-Polyole und / oder Polyetherpolyole enthalten.

7. Polyharnstoff-System nach einem der Ansprüche 1 bis 6, **dadurch gekennzeichnet, dass** die Polyole A2) ein zahlenmittleres Molekulargewicht von 4000 bis 8500 g/mol aufweisen.

8. Polyharnstoff-System nach einem der Ansprüche 1 bis 7, **dadurch gekennzeichnet, dass** es als Komponente C) organische Füllstoffe umfasst.

9. Polyharnstoff-System nach Anspruch 8, **dadurch gekennzeichnet, dass** die organischen Füllstoffe hydroxyfunktionelle Verbindungen sind.

10. Polyharnstoff-System nach Anspruch 9, **dadurch gekennzeichnet, dass** die hydroxyfunktionellen Verbindungen Polyetherpolyole sind.

11. Polyharnstoff-System nach Anspruch 9 oder 10, **dadurch gekennzeichnet, dass** die hydroxyfunktionellen Verbindungen eine mittlere OH-Funktionalität von 1,5 bis 3 aufweisen.

12. Polyharnstoff-System nach einem der Ansprüche 1 bis 11, **dadurch gekennzeichnet, dass** es als Komponente D) pharmakologisch aktive Verbindungen umfasst.

13. Polyharnstoff-System nach einem der Ansprüche 1 bis 12 zum Verschließen, Verbinden, Verkleben oder Abdecken von Zellgewebe, zur Stillung des Austritts von Blut oder Gewebeflüssigkeiten oder dem Verschluss von Leckagen in Zellgewebe.

14. Dosiersystem mit zwei Kammern für ein Polyharnstoff-System nach einem der Ansprüche 1 bis 13, **dadurch gekennzeichnet, dass** in der einen Kammer die Komponente A) und in der anderen Kammer die Komponenten B) sowie gegebenenfalls die Komponenten C), und D) des Polyharnstoff-Systems enthalten sind.

## Claims

1. Polyurea system comprising
as component A) isocyanate-functional prepolymers obtainable from reacting aliphatic isocyanates A1) with polyols A2),
as component B) amino-functional compounds of general formula (I) 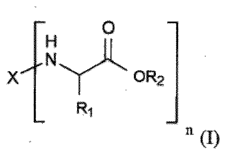 wherein
X represents an organic group containing a tertiary amino functionality that does not contain a Zerewitinoff-active hydrogen,
R₁ represents a CH₂-COOR₃ group wherein R3 represents an organic group that does not contain a Zerewitinoff active hydrogen, a linear or branched C₁ to C₄ alkyl, a cyclopentyl, a cyclohexyl group or H,
R₂ represents an organic group that does not contain a Zerewitinoff active hydrogen,
n represents an integer of 2 or 3

2. Polyurea system according to claim 1, wherein X represents a group of formula (II) 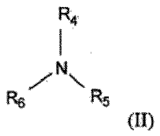 wherein
R₄, R₅, R₆ each independently of one another represent an organic group that does not contain a Zerewitinoff active hydrogen.

3. Polyurea system according to claim 2, wherein R₄, R₅, R₅ each independently of one another or simultaneously, are linear or branched.

4. Polyurea system according to claim 3, wherein R₄, R₅, R₆ each independently of one another or simultaneously represent a methyl, ethyl, propyl or butyl group, wherein at least one of groups R₄, R₅, R₆ represents a methylene, ethylene, propylene or butylene group.

5. Polyurea system according to any one of claims 1 to 4, wherein groups R1, R2 and optionally R3 are each independently of one another, or simultaneously, a linear or branched C1 to C10.

6. Polyurea system according to any one of claims 1 to 5, wherein polyols A2) contain polyester polyols and/or polyester-polyether polyols and/or polyether polyols.

7. Polyurea system according to any one of claims 1 to 6, wherein polyols A2) have a number average molecular weight of 4000 to 8500 g/mol.

8. Polyurea system according to any one of claims 1 to 7, wherein as component C) it contains organic fillers.

9. Polyurea system according to claim 8, wherein the organic fillers are hydroxy-functional compounds.

10. Polyurea system according to claim 9 wherein the hydroxyl-functional compounds are polyether polyols.

11. Polyurea system according to any one of claim 9 or 10, wherein the hydroxyl-functional compounds have an average OH functionality of 1.5 to 3.

12. Polyurea system according to any one of claims 1 to 11, wherein as component D) it contains pharmacologically active compounds.

13. Polyurea system according to any one of claims 1 to 12 for sealing, bonding, gluing, or covering of tissue, for stemming the leakage of blood or tissue fluids, or for the sealing of leaks in tissue.

14. Dosing system consisting of two chambers for a polyurea system according to one of claims 1 to 13, wherein one chamber contains component A) and the other chamber contains components B) and, optionally, components C) and D) of the polyurea system.

## Revendications

1. Système de polyurée comprenant
comme composant A) des prépolymères fonctionnels isocyanate pouvant être obtenus par transformation d'isocyanates aliphatiques A1) avec des polyols A2),
comme composant B) des composés aminofonctionnels de formule générale (I) 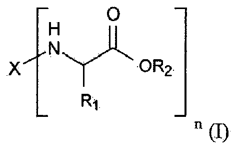 où
X est un groupe fonctionnel organique comportant une fonction amino tertiaire ne présentant aucun atome d'hydrogène actif de Zerewitinoff,
R₁ est un groupe fonctionnel CH₂-COOR₃ où R₃ est un groupe fonctionnel organique ne présentant aucun atome d'hydrogène actif de Zerewitinoff, un groupe fonctionnel alkyle en C1 à C4 linéaire ou ramifié, un groupe fonctionnel cyclopentyle, un groupe fonctionnel cyclohexyle ou H,
R₂ est un groupe fonctionnel organique ne présentant aucun atome d'hydrogène actif de Zerewitinoff,
n correspond à 2 ou 3.

2. Système de polyurée selon la revendication 1, **caractérisé en ce que** X est un
groupe fonctionnel de formule (II) 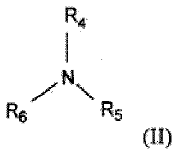 où
R₄, R₅, R₆ sont chacun, indépendamment l'un de l'autre, des groupes fonctionnels organiques ne présentant aucun atome d'hydrogène actif de Zerewitinoff.

3. Système de polyurée selon la revendication 2, **caractérisé en ce que** R₄, R₅, R₆ sont chacun, indépendamment l'un de l'autre ou simultanément, des groupes fonctionnels organiques linéaires ou ramifiés.

4. Système de polyurée selon la revendication 3, **caractérisé en ce que** R₄, R₅, R₆ sont chacun, indépendamment l'un de l'autre ou simultanément, des groupes fonctionnels méthyle, éthyle, propyle ou butyle, au moins un des groupes fonctionnels R₄, R₅, R₆ étant un groupe fonctionnel méthylène, éthylène, propylène ou butylène.

5. Système de polyurée selon l'une des revendications 1 à 4, **caractérisé en ce que** les groupes fonctionnels R₁, R₂ et, le cas échéant, R₃ sont chacun, indépendamment l'un de l'autre ou simultanément, des groupes fonctionnels organiques en C1 à C10 linéaires ou ramifiés.

6. Système de polyurée selon l'une des revendications 1 à 5, **caractérisé en ce que** les polyols A2) comportent des polyesterpolyols et/ou des polyester-polyétherpolyols et/ou des polyétherpolyols.

7. Système de polyurée selon l'une des revendications 1 à 6, **caractérisé en ce que** les polyols A2) présentent un poids moléculaire moyen en nombre de 4000 à 8500 g/mol.

8. Système de polyurée selon l'une des revendications 1 à 7, **caractérisé en ce qu'**il comprend comme composant C) des matières de charge organiques.

9. Système de polyurée selon la revendication 8, **caractérisé en ce que** les matières de charge organiques sont des composés hydroxyfonctionnels.

10. Système de polyurée selon la revendication 9, **caractérisé en ce que** les composés hydroxyfonctionnels sont des polyétherpolyols.

11. Système de polyurée selon la revendication 9 ou 10, **caractérisé en ce que** les composés hydroxyfonctionnels présentent une fonctionnalité OH moyenne de 1,5 à 3.

12. Système de polyurée selon l'une des revendications 1 à 11, **caractérisé en ce qu'**il comprend comme composant D) des composés pharmacologiquement actifs.

13. Système de polyurée selon l'une des revendications 1 à 12 destiné à fermer, lier, agglutiner ou recouvrir des tissus cellulaires dans le but de stopper une hémorragie ou l'écoulement de fluides tissulaires ou de sceller des fuites dans les tissus cellulaires.

14. Système de dosage à deux chambres pour un système de polyurée selon l'une des revendications 1 à 13, **caractérisé en ce que** le composant A) est contenu dans ladite première chambre et les composants B) sont contenus dans ladite seconde chambre, ainsi que, le cas échéant, les composants C) et D) du système de polyurée.
